**Europäisches Patentamt**

**European Patent Office**

**Office européen des brevets**

(19)

(11) Publication number : **0 358 373 B1**

(12) # EUROPEAN PATENT SPECIFICATION

(45) Date of publication of patent specification :
**26.08.92 Bulletin 92/35**

(51) Int. Cl.[5] : **A61F 6/06**

(21) Application number : **89308526.6**

(22) Date of filing : **23.08.89**

(54) Intravaginal device.

(30) Priority : **06.09.88 US 240805**

(43) Date of publication of application :
**14.03.90 Bulletin 90/11**

(45) Publication of the grant of the patent :
**26.08.92 Bulletin 92/35**

(84) Designated Contracting States :
**AT BE CH DE ES FR GB GR IT LI LU NL SE**

(56) References cited :
**WO-A-88/05291**
**CH-A- 145 262**
**FR-A- 2 606 275**
**US-A- 4 004 591**

(73) Proprietor : **Quiroz , Roberto**
**341 Lucero Lane**
**El Paso Texas 79917 (US)**

(72) Inventor : **Quiroz , Roberto**
**341 Lucero Lane**
**El Paso Texas 79917 (US)**

(74) Representative : **Harrison, Philippa Dinah et al**
**A. A. THORNTON & CO Northumberland**
**House 303-306 High Holborn**
**London WC1V 7LE (GB)**

EP 0 358 373 B1

## Description

This invention relates to an intravaginal device.

Mankind is sexually active. Although artificial means for reproduction have been found, mankind's existence still depends on his sexual reproductive activities. Mankind has experienced a population explosion. He has also experienced an explosion in communicable diseases. Some of these diseases are deadly. One such disease Acquired Immunity Deficiency Syndrome is at this time spreading at an alarming rate and is incurable. The device presented here is to aid in the prevention of the spread of venereal diseases. The device is similar to the condom, but it also differs from the condom in many respects. The device is made of the same elastic materials as the materials used to make the condom. The condom is physically designed, and used, to cover the male penis. And its main function is to prevent lesions, infections and fluids on and from the penis from coming in contact with the female. The surface area covered by the condom is small. The high rate of breakage and failure of the condom has been widely reported.

It has been proposed, in WO-A-8805291, to provide a barrier device for insertion into the female's vagina prior to intercourse. Such a device, generally known as a "female condom", takes the form of a sheath, broadly similar to a conventional male condom, but is additionally provided with means for securing it in position on the female user's body.

Female condoms of the type of WO-A-8805291 can suffer from the same problem as do conventional male condoms, namely the breakage or failure of the single layer of material.

According to the present invention there is provided an intravaginal device comprising an elongated and elastic bag, one end of said bag being adapted to fit into a female vagina and cover the surface of the vagina during sexual intercourse, characterized in that said bag is a closed bag, and that a second end of said bag is the size and shape of a standard condom, and is arranged to extend initially outside of the vagina and to cover a penis with its exterior surface as the penis enters the vagina.

The double-ended arrangement of the device of the present invention provides extra safety. Should the material of one of the ends of the bag break during intercourse, the material of the other bag will still prevent contact between the penis and the vagina, preventing infection.

The bag may additionally be provided with an upper portion and a lower portion to cover the external genitalia, with an elastic band being provided round the perimeters of said upper and lower portions.

Means may be provided for holding and supporting the bag in place during use.

Further features and advantages of the present invention will now be described, by way of example only, with reference to the accompanying drawings in which :

Fig. 1 is a perspective view of a first embodiment of the device of the present invention; and

Fig. 2 is a perspective view of a further embodiment of device in accordance with the present invention, including means for covering the external genitalia.

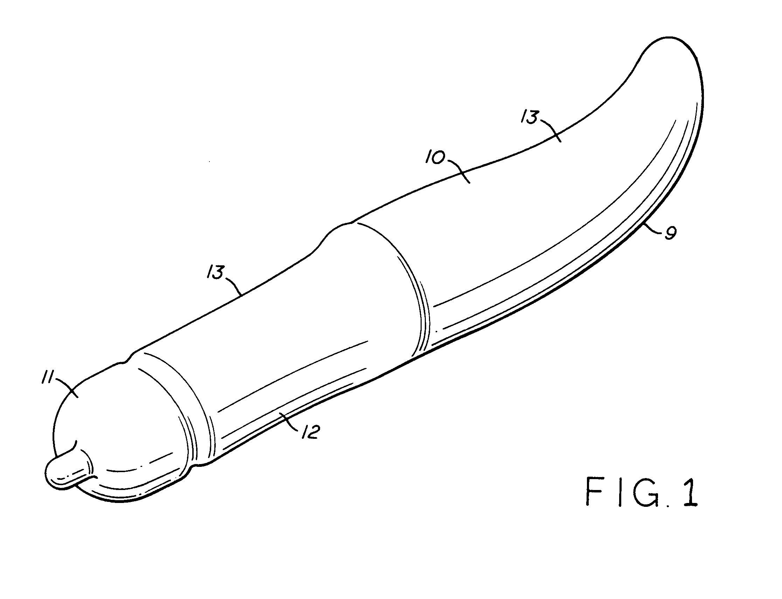

The device shown in Fig. 1 comprises an elongate, elastic bag having a shape similar to a figure eight. Each end of the bag is an elongated bulb. One end 9 is adapted to fit into a female vagina and cover, with its external surface 10, the surface of the vagina during sexual intercourse. The other end 11 is smaller and is the shape and size of a standard condom. The penis in penetrating the vagina comes into contact with the exterior surface 12 of the smaller elongated bulb. This exterior surface 12 covers the penis as it enters into the vagina. The penis pushes the interior surface into the larger bulb. When the penis is in the vagina its surface is covered by the exterior surface 12 of the small bulb and the inner surface of the vagina is covered by the exterior surface 10 of the larger bulb. As a result two layers of the elastic material of the device separate the penis and its fluids from the inner surface of the vagina. No means for holding the device in place are shown.

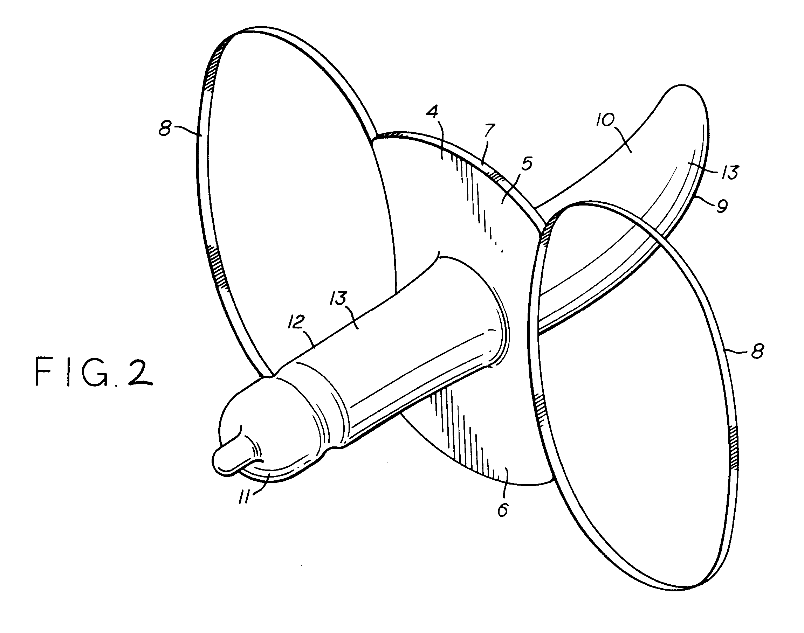

Fig. 2 shows the device described in Fig. 1 with additional coverings 4 and straps 8. The coverings 4 for the external genitalia comprise an upper covering 5 and a lower covering 6, an elastic band 7 running around the perimeter of the coverings. The straps 8 are intended to support and hold the device in place. A leg is intended to fit into each strap 8, which is then pulled up over the leg. Both versions of the device come with lubricating jellies 13.

## Claims

1. An intravaginal device comprising an elongated and elastic bag, one end (9) of said bag being adapted to fit into a female vagina and cover the surface of the vagina during sexual intercourse, characterized in that said bag is a closed bag, and that a second end (11) of said bag is the size and shape of a standard condom, and is arranged to extend initially outside of the vagina and to cover a penis with its exterior surface (12) as the penis enters the vagina.

2. An intravaginal device as claimed in Claim 1, wherein said bag is provided with an upper portion (5) and a lower portion (6) to cover the external genitalia, and an elastic band (7) is provided around the perimeters of said upper and lower portions (5,6).

3. An intravaginal device as claimed in Claim 1 or Claim 2, comprising a means (8) for holding and supporting said bag in place.

4. An intravaginal device as claimed in Claim 3, when dependent on Claim 2, wherein the means (8) for holding and supporting said bag in place is arranged to hold said upper and lower portions in place.

**Patentansprüche**

1. Eine Intravaginalvorrichtung, welche einen länglichen und elastischen Beutel umfaßt, wobei ein Ende (9) des Beutels dazu vorgesehen ist, in eine weibliche Scheide eingebracht zu werden und die Oberfläche der Scheide während eines Geschlechtsverkehrs zu bedecken, **dadurch gekennzeichnet**, daß der Beutel ein geschlossener Beutel ist, und daß ein zweites Ende (11) des Beutels das Format und die Form eines Standardkondoms aufweist, und dazu vorgesehen ist, sich anfänglich außerhalb der Scheide zu erstrecken und einen Penis mit seiner äußeren Oberfläche (12) zu bedecken, wenn der Penis in die Scheide eintritt.

2. Eine Intravaginalvorrichtung nach Anspruch 1, wobei der Beutel mit einem oberen Teil (5) und einem unteren Teil (6) zum Abdecken der äußeren Genitalien versehen ist, und ein elastisches Band (7) um die Peripherien des oberen und unteren Teils (5, 6) herum vorgesehen ist.

3. Eine Intravaginalvorrichtung nach Anspruch 1 oder 2, welche eine Einrichtung (8) zum Halten und Abstützen des Beutels an Ort und Stelle umfaßt.

4. Eine Intravaginalvorrichtung nach Anspruch 3 in Verbindung mit Anspruch 2, wobei die Einrichtung (8) zum Halten und Abstützen des Beutels an Ort und Stelle dazu vorgesehen ist, den oberen und unteren Teil an Ort und Stelle zu halten.

**Revendications**

1. Dispositif intravaginal comprenant une poche élastique et allongée, une extrémité (9) de ladite poche étant conçue pour s'insérer dans un vagin féminin et couvrir la surface du vagin durant l'acte sexuel, caractérisé en ce que ladite poche est une poche fermée et en ce qu'une seconde extrémité (11) de ladite poche a la taille et la forme d'un préservatif classique et est disposée de façon à s'étendre initialement à l'extérieur du vagin et à couvrir un pénis par sa surface externe (12) lorsque ce dernier pénètre dans le vagin.

2. Dispositif intravaginal selon la revendication 1, dans lequel ladite poche est munie d'une partie supérieure (5) et d'une partie inférieure (6) pour couvrir les organes génitaux externes et en ce qu'une bande élastique (7) est prévue sur les périmètres desdites parties supérieure et inférieure (5, 6).

3. Dispositif intravaginal selon la revendication 1 ou la revendication 2, comprenant un moyen (8) pour fixer et maintenir en place ladite poche.

4. Dispositif intravaginal selon la revendication 3, lorsqu'elle est dépendante de la revendication 2, dans lequel le moyen (8) de fixation et maintien en place de ladite poche est disposé de façon à maintenir en place lesdites parties supérieure et inférieure.

FIG. 1

FIG. 2

EP 0 358 373 B1